# EUROPEAN PATENT SPECIFICATION

(11) **EP 2 646 421 B1**
(45) Date of publication and mention of the grant of the patent: **06.05.2015**
(21) Application number: 11801632.8
(22) Date of filing: 29.11.2011
(51) Int. Cl.: C07D 243/24, C07D 243/26, A61K 31/5513, A61P 9/10, A61P 25/28

(54) **7-NITRO-5-PHENYL-1-(PYRROLIDIN-1-YLMETHYL)-1H-BENZO[E][1,4]DIAZEPIN-2(3H)-ONE AND OTHER BENZODIAZEPINE DERIVATIVES**
7-NITRO-5-PHENYL-1- (PYRROLIDIN-1-YLMETHYL) -1H-BENZO [E] [1,4] DIAZEPIN-2 (3H) -ON UND ANDERE BENZODIAZEPINDERIVATE
7-NITRO-5-PHÉNYL-1-(PYRROLIDIN-1-YLMÉTHYL)-1H-BENZO[E][1,4]DIAZÉPIN-2(3H)-ONE ET AUTRES DÉRIVÉS DE BENZODIAZÉPINE

(30) Priority: 30.11.2010 IT FI20100233
(43) Date of publication of application: 09.10.2013
(73) Proprietor: Pignataro, Giuseppe, 80078 Pozzuoli (IT); Annunziato, Lucio, 80122 Napoli (IT); Molinaro, Pasquale, 81100 Caserta (IT); Scorziello, Antonella, 80013 Casalnuovo Di Napoli (IT); Secondo, Agnese, 80078 Pozzuoli (IT); Pannaccione, Anna, 80122 Napoli (IT); Cuomo, Ornella, 80128 Napoli (IT); Cantile, Maria, 81030 San Marcellino (IT); Di Renzo, Gianfranco, 80131 Napoli (IT); Caliendo, Giuseppe, 80034 Marigliano (IT); Santagada, Vincenzo, 87070 Cerchiara Di Calabria (IT); Severino, Beatrice, 80018 Mugnano Di Napoli (IT); Fiorino, Ferdinando, 82100 Benevento (IT)
(72) Inventor: Pignataro, Giuseppe, 80078 Pozzuoli (IT); Annunziato, Lucio, 80122 Napoli (IT); Molinaro, Pasquale, 81100 Caserta (IT); Scorziello, Antonella, 80013 Casalnuovo Di Napoli (IT); Secondo, Agnese, 80078 Pozzuoli (IT); Pannaccione, Anna, 80122 Napoli (IT); Cuomo, Ornella, 80128 Napoli (IT); Cantile, Maria, 81030 San Marcellino (IT); Di Renzo, Gianfranco, 80131 Napoli (IT); Caliendo, Giuseppe, 80034 Marigliano (IT); Santagada, Vincenzo, 87070 Cerchiara Di Calabria (IT); Severino, Beatrice, 80018 Mugnano Di Napoli (IT); Fiorino, Ferdinando, 82100 Benevento (IT)
(74) Representative: Valenza, Silvia
(86) International application number: PCT/EP2011/071252
(87) International publication number: WO 2012/072620

(56) References cited:
- WO-A2-03/106628
- Cantile et al.: "Neurounina, a novel compound that increases Na+/Ca2+ exchanger activity, protects against stroke damage", FENS Abstr., vol. 5, 016.4, 4 July 2010 (2010-07-04), 2010, XP002634140, Retrieved from the Internet: URL:http://fens2010.neurosciences.asso.fr/ abstracts/rpdf1/a016_4.pdf [retrieved on 2011-04-26]
- G. PIGNATARO ET AL.: "Evidence for a protective role played by the Na+/Ca2+ exchanger in cerebral ischemia induced by middle cerebral artery occlusion in male rats", NEUROPHARMACOLOGY, vol. 46, 2004, pages 439-448, XP002634141,
- A. J. HANNAN: "European Neuroscience- Seventh Biennial FENS Forum", IDRUGS, vol. 13, no. 9, 2010, pages 607-609,

## Description

### FIELD OF THE INVENTION

The present invention relates to the field of heterocyclic compounds, in particular to benzodiazepine derivatives and relates to a method for the synthesis thereof.The compounds of the invention are useful for medical use, particularly as drugs active in the treatment of cerebral ischemia and of other diseases characterized by the dysregulation of sodium and calcium homeostasis, such as amyotrophic lateral sclerosis, Alzheimer's disease, Parkinson's disease, and renal or cardiac ischemia.

### STATE OF THE ART

Stroke is the third leading cause of death and the leading cause of severe disabilities in the developed world and implies relevant costs for the healthcare system.

Recombinant tissue-type plasminogen activator (rtPA) is a thrombolytic agent currently the only used drug in clinical practice for the treatment of peripheral and cerebral ischemia; however, the overall clinical benefit this treatment has been limited by the short therapeutic window and by its intrinsic neurotoxicity, because this drug could permeate through hematoencephalic barrier following stroke. For these reasons there is a great interest in developing alternative pharmacological approaches. Recently, the Sodium/Calcium Exchanger (NCX) has emerged as one of the most promising molecular target.

It has been demonstrated the importance of this exchanger during cerebral ischemia. In fact the lack of oxygen and glucose causes a reduction in the levels of ATP, determining the blockade of the two main ATP-dependent transmembrane transporters: Na⁺/K⁺ ATPase and Ca²⁺ ATPase.

Consequently, there is a loss of homeostasis of these ions with a progressive increase in their intracellular concentrations. This effect causes anoxia and neuronal damage. Under these conditions, the role played by NCX -that does not require ATP to maintain sodium and calcium concentrations within physiological levels- assumes a greater relevance.

In fact, in the early phase of neuronal anoxic insult, the Na⁺-K⁺ ATPase blockade increases [Na⁺]ᵢ, which in turn, induces the Na⁺/Ca²⁺ exchanger in the reverse mode of operation. Although NCX increases [Ca²⁺]ᵢ in this phase, its effect could be beneficial for neurons, because it contributes to a decrease in [Na⁺]ᵢ overload, thus preventing cell swelling and death. Conversely, in the later phase of neuronal anoxia, when [Ca²⁺]ᵢ overload takes place, NCX forward mode of operation contributes to the lowering of [Ca²⁺]ᵢ, thus protecting neurons from [Ca²⁺]ᵢ-induced neurotoxicity.

Thus NCX might be a possible drug target for treating cerebral ischemia and some serious diseases characterized by loss of control of the ionic homeostasis, such as Alzheimer's, Parkinson's, multiple sclerosis, amyotrophic lateral sclerosis and epilepsy. In addition, NCX can be an important drug target for treating cardiac and renal ischemia.

The Na⁺/Ca²⁺ exchanger (NCX) is an important bi-directional transporter that couples 3 Na⁺ ions and 1 Ca²⁺ ion. It has been identified three isoforms of this antiporter with a different sensibility to ATP levels. NCX, under physiological conditions, couples the extrusion of one Ca²⁺ to the influx of 3 Na⁺ ions into the cells, working in the "*forward mode*", but if the electrochemical gradients reverts, NCX could extrude of 3 Na⁺ ions and influx of one Ca²⁺ ion, working in the "*reverse mode*". An increased activity of this exchanger could limit or counteract the excess of intercellular Ca²⁺ and Na⁺, and consequently, leads to cellular survival. For these reasons, NCX was highlighted as a potential molecular target for the development of new compound to be utilized in stroke treatment. Therefore, in the last years, scientific interest has been focused to find new compounds that increase NCX activity, but nevertheless the great effort, to date it has been identified only NCX inhibitors that are not selective for each NCX isoforms. Therefore, the purpose of the present invention is to provide new compounds which act as specific and selective activators for each isoform of NCX.

### SUMMARY OF THE INVENTION

Subject-matter of the present invention are compounds of formula (I): 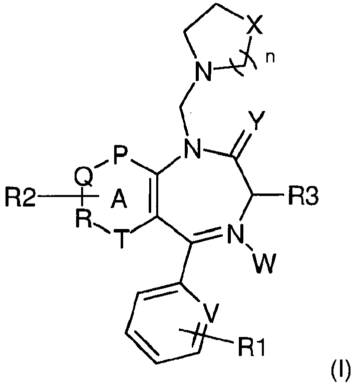 wherein
ring A is a 5- or 6-membered aromatic or heteroaromatic ring, and therefore a group chosen from P, Q, R or T may be absent; P, Q, R and T independently of one another are chosen from CH (or C is substituted by R2), N, O and S;
R1 and R2 independently of one another are chosen from hydrogen, halogen, Ak, O-Ak, Ph, O-Ph, Ph-Ak, heteroaryl, CF₃, OCF₃, NO₂, NH₂, NH-Ak, NAk₂, COOAk, S-Ak, SO₂-Ak, CN;
R3 is chosen from hydrogen, halogen, Ak, OAk, O-CO-Ak, OH;
Y is either O or S;
W is O, if present;
V is chosen between CH or C if substituted by R1, and N;
X is chosen between CH₂, O, NH, NAk and n = 1,2,3;
wherein Ak means alkyl.

In the compounds of formula (I), optical isomers, enantiomers or diastereoisomers and mixtures thereof, and pharmaceutically acceptable organic and inorganic salts are intended to be included.

A compound of formula (I), in this case the formula (Ia), hereinafter called neurounina-1, resulted as a selective activator of NCX1 and NCX2. Furthermore, neurounina-1 has been shown to have neuroprotective properties in models of cerebral ischemia, *in vitro* and *in vivo*, on cortical neurons. Thus compounds of formula (I) are useful for use in the treatment of cerebral ischemia or any other disease characterized by the derangement of sodium and calcium ions homeostasis, such as amyotrophic lateral sclerosis, Alzheimer's and Parkinson's disease, renal and cardiac ischemia, and other neurodegenerative diseases, but also for use in the treatment of anxiety, sedation, insomnia and striatum muscle spasms.

Subject-matter of the present invention are also pharmaceutical compositions comprising a compound of formula (I) and at least another pharmaceutically acceptable ingredient.

Further subject-matter of the present inventionis a method for synthesizing a compound of formula (I) starting from a compound of formula (II) and a compound and formula (III): 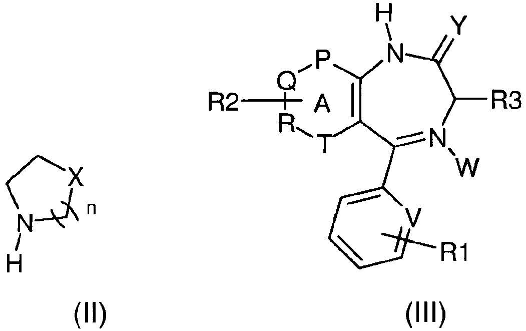 wherein R1, R2, R3, the A ring, P, Q, R, T, X, Y, V, W and n are as described above.

### DESCRIPTION OF FIGURES

**Figure 1****: A.** Chemical structure of nitro-benzodiazepine derivative (neurounina-1). **B.** Dose-response curves of the compound neurounina-1 on forward and reverse activity of NCX.
**Figure 2****:** Microfluorimetry analysis of 10 nM neurounina-1 effects on the three NCX isoforms.
**Figure 3****:** Electrophysiological analysis of neurounina-1 effect on the three NCX isoforms.
**Figure 4****:** Effect of 10 nM neurounina-1 on NCX1/NCX3 chimeras measured by Na⁺ᵢ-dependent ⁴⁵Ca²⁺ Uptake and Efflux
**Figure 5****: A.** Amino acid alignment of alpha1 and alpha2 regions responsible for drug sensitivity between NCX1 and NCX3. Mutated amino acids are indicated in bold. Asterisks indicate the position of mutations that alters the neurounina-1 sensitivity. **B.** Effect of neurounina-1 on NCX1 mutants measured by Na⁺ₒ-dependent ⁴⁵Ca²⁺ uptake.
**Figure 6****:** Effect of neurounina-1 on NCX1 mutants measured by Fura-2 Ca²⁺ monitored increase.
**Figure 7****:** Effect of neurounina-1 on mitochondrial activity and on cell survival in stably transfected NCX-BHK cells and in primary cortical neurons.
**Figure 8****:** Evaluation of neurounina-1 effect on ischemic damage in mice subjected to tMCAO.

### DETAILED DESCRIPTION

Subject.matter of the invention are compounds of formula (I): 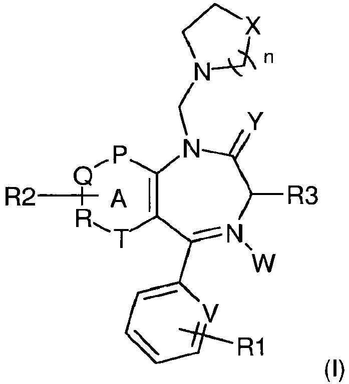 as described above.

Preferably X is CH₂ and *n* is 1 or 2.

Preferably the A ring is benzene and R2 is Cl, NO₂ or NH₂.

Preferably V is CH or C if substituted by R1 and R1 is H or Cl. Preferably R3 is H or OH.

Preferably Y is O.

Preferably W is absent.

The compounds of this invention can be salified with pharmaceutically acceptable acids such as hydrochloric, citric, fumaric, maleic, trifluoracetic acid, etc.. Preferred compounds of formula (I) are those in which:
X is CH₂ and *n* is 1 or 2,
the A ring is benzene,
R1 is H or Cl
R2 is Cl, NO₂ or NH₂,
R3 is H or OH,
YisO,
V is CH or C if substituted by R1,
W is absent.

In particular, are preferred compounds of formula (Ia) or (Ib)

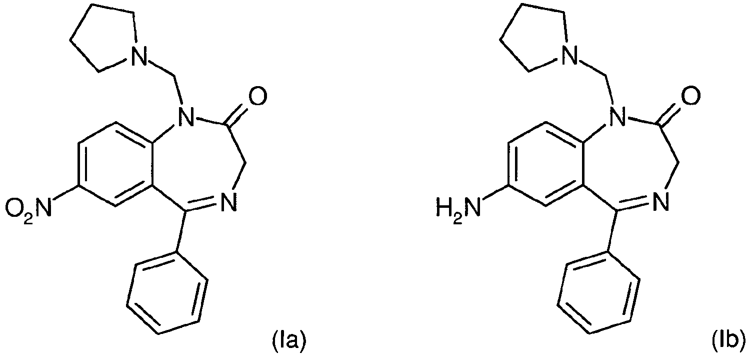

The compound (Ia) is preferred and is a nitrobenzodiazepine derivative, in particular is 7-nitro-5-phenyl-1(pyrrolidin-1-ylmethyl)-1H-benzo[e][1,4] diazepin-2(3H)-one, and its pharmaceutically acceptable salts. In particular, it is preferred the compound of formula (Ia) as trifluoracetic salt, called neurounina-1, which is the first compound that increases NCX1 and NCX2 activity.

We investigated the effect of neurounina-1 on NCX1, NCX2 and NCX3 activity in forward and reverse mode of operation by means of patch clamp, single-cell Fura-2 microfluorimetry and ⁴⁵Ca²⁺ radiotracer fluxes techniques. The results showed that this compound increases, in a dose-dependent manner, NCX1 and NCX2 activity, but it did not exert any effect on NCX3 activity. Moreover, it is useful to consider that NCX1 and NCX2 are sensitive to a reduction in the levels of intracellular ATP, unlike that of NCX3 was not sensitive to this nucleotide. From the functional assays it resulted that neurounina-1 showed EC50 su NCX1 e NCX2 comprised between 1 nM and 2 nM, from which it resulted to be the most potent compound on NCX1 and NCX2.

In addition, we identified molecular determinants of this compounds on NCX1 responsible for the difference in the drug sensitivity of isoforms. This information could help to understand the mechanism of action of drugs that act on NCX to synthesize, in a rational way, new molecules that have more power, efficiency and selectivity.

Once the pharmacological properties of neurounina-1 have been clarified, we examined the putative toxic effect of neurounina-1. And results did not show any toxic effect on cell death and on mitochondrial dysfunction even exposure up to micromolar concentrations, almost 10.000 times greater than EC₅₀. Moreover, since NCX activity is has been demonstrated to be involved in cerebral ischemia, we examined the putative protective effects of neurounina-1 on *in vitro* and *in vivo* experimental models of cerebral ischemia and the results showed that the compound has a significant neuroprotective activity.

In conclusion, the compound neurounina-1 increases the activity of NCX1 and NCX2. The intraperitoneal administration of this drug in mice subjected to cerebral ischemia decreases the ischemic volume.

Therefore we propose, a medical use of this compound as as a new neuroprotective drug potentially useful for the treatment of (against) stroke and (in) other diseases characterized by the deregulation of sodium and calcium homeostasis.

Compounds of formula (I) can be prepared by reacting a compound of formula (II) 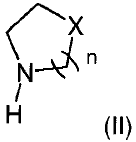 Wherein X is chosen from CH₂, O, NH, NAk and n = 1,2,3;
with formaldehyde;
then this mixture is reacted with a compound of formula (III): 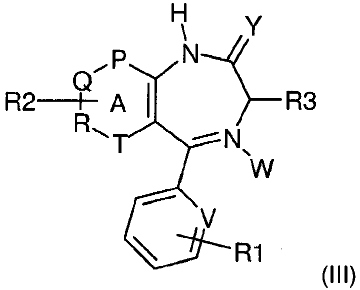 wherein
A ring is a 5- or 6-membered aromatic or heteroaromatic ring, and therefore a group chosen from P, Q, R or T may be absent; P, Q, R and T independently of one another are chosen from CH (or C if substituted by R2), N, O and S;
R1 and R2 independently of one another are chosen from hydrogen, halogen, Ak, O-Ak, Ph, O-Ph, Ph-Ak, heteroaryl, CF₃, OCF₃, NO₂, NH₂, NH-Ak, NAk₂, COOAk, S-Ak, S02-Ak, CN;
R3 is chosen from hydrogen, halogen, Ak, OAk, O-CO-Ak, OH;
Y is chosen from O and S;
W is O, if present;
V is chosen from CH or C if substituted by R1, and N;
X is chosen from CH2, O, NH, NAk and n = 1,2,3;
wherein Ak means alkyl;
Neurounia-1 was synthesized by a process comprising the following steps:
- reaction of formaldehyde with pyrrolidine
- reaction of nitrazepam with the mixture obtained from the previous step.

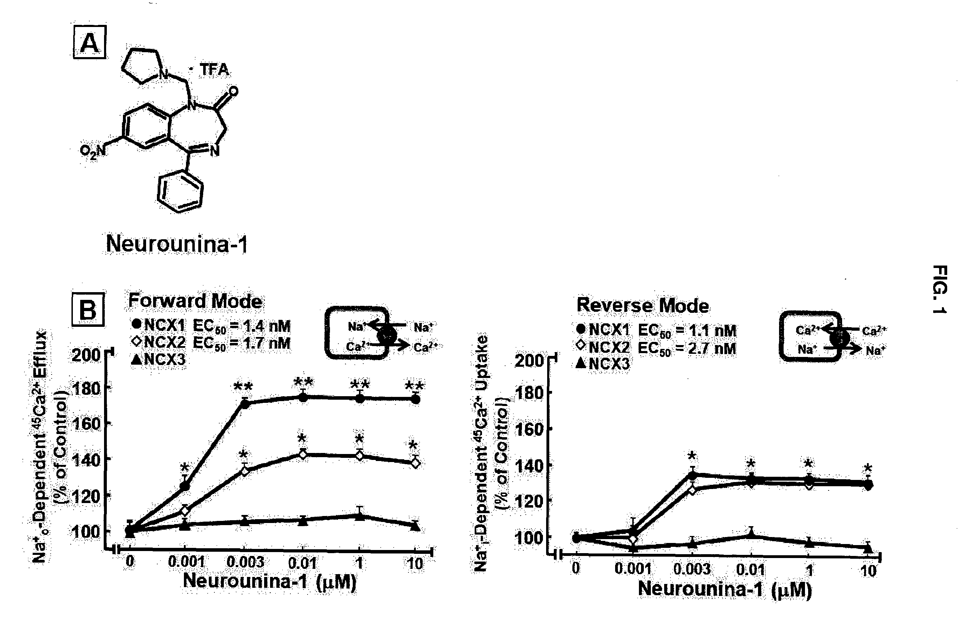

Once synthesized the compound called neurounina-1, has been evaluated the effect of this nitro-benzodiazepine derivative on NCX in forward and reverse mode of operation, by means of patch-clamp, single-cell Fura-2 microfluorimetry, and ⁴⁵Ca²⁺ radiotracer fluxes techniques. The first characterization of the neurounina-1 activity on the Na⁺/Ca²⁺ exchanger in both forward and reverse mode of operation was performed by studying the efflux and influx of the ⁴⁵Ca²⁺ radiotracer. By means of Naᵢ⁺-dependent ⁴⁵Ca²⁺ uptake technique we observed that neurounina-1 increased the forward mode of NCX1 and NCX2 activity by 70% and 30%, respectively, with an EC₅₀ of 1.4 nM for NCX1 and 1.7 nM for NCX2, but it did not significantly affect the activity of NCX3. Regarding the reverse mode activity of NCX, we observed that neurounina-1 increase both the NCX1 (EC₅₀ = 1.1 nM) and NCX2 (EC₅₀= 2.7 nM) activities by 40%, whereas it did not affect the activity of NCX3 (Fig. 1B).

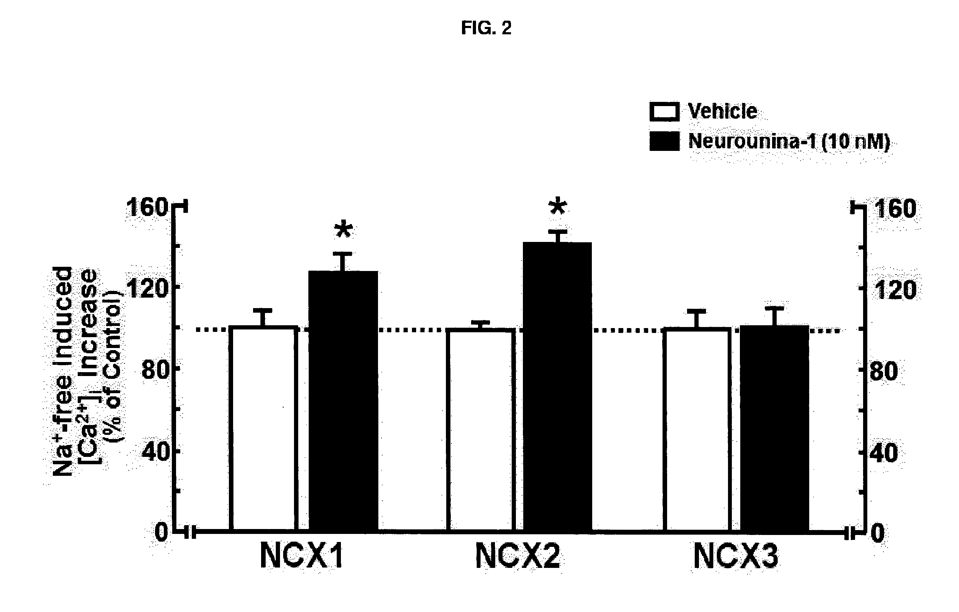

The effect of neurounina-1 on NCX, measured with Fura-2 AM single-cell computer-assisted video imaging technique, showed an increased reverse mode activity of NCX1 and NCX2 by 30% and 40%, respectively, whereas BHK cells expressing NCX3 did not show any significant difference in the activity of Na⁺/Ca²⁺ exchange (Fig 2).

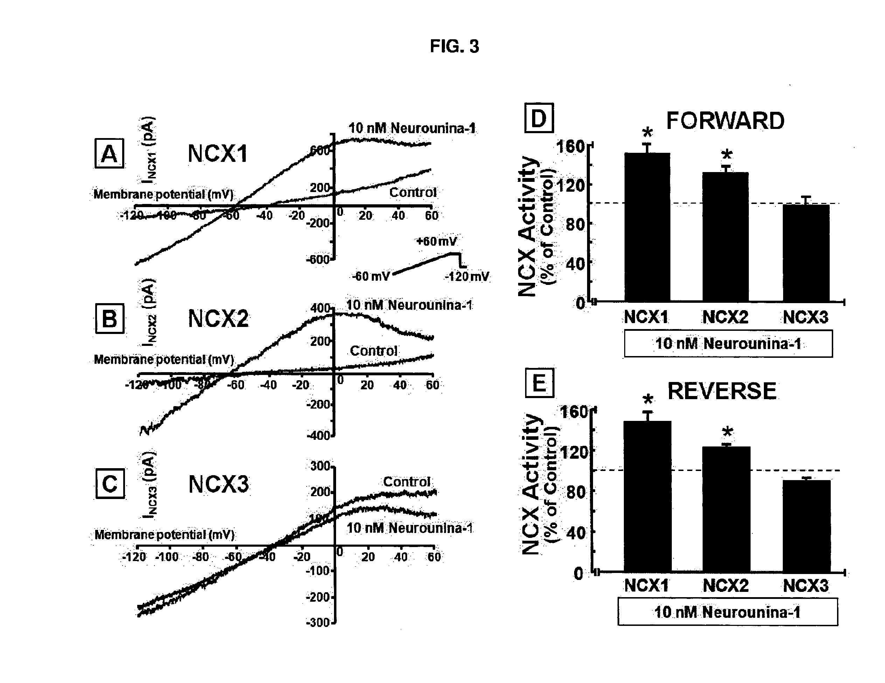

To further investigate the effects of this compound on the Na⁺/Ca²⁺ exchanger activity, we performed electrophysiological experiments on BHK cells stably transfected with each of NCX isoforms (named BHK-NCX1, BHK-NCX2 and BHK-NCX3). BHK-NCX1 and BHK-NCX2 cells exposed to 10 minutes with a solution containing 10 nM neurounina-1 showed an increased forward and reverse activity of both NCX1 and NCX2 by 60% and 30%, respectively (Fig. 3).

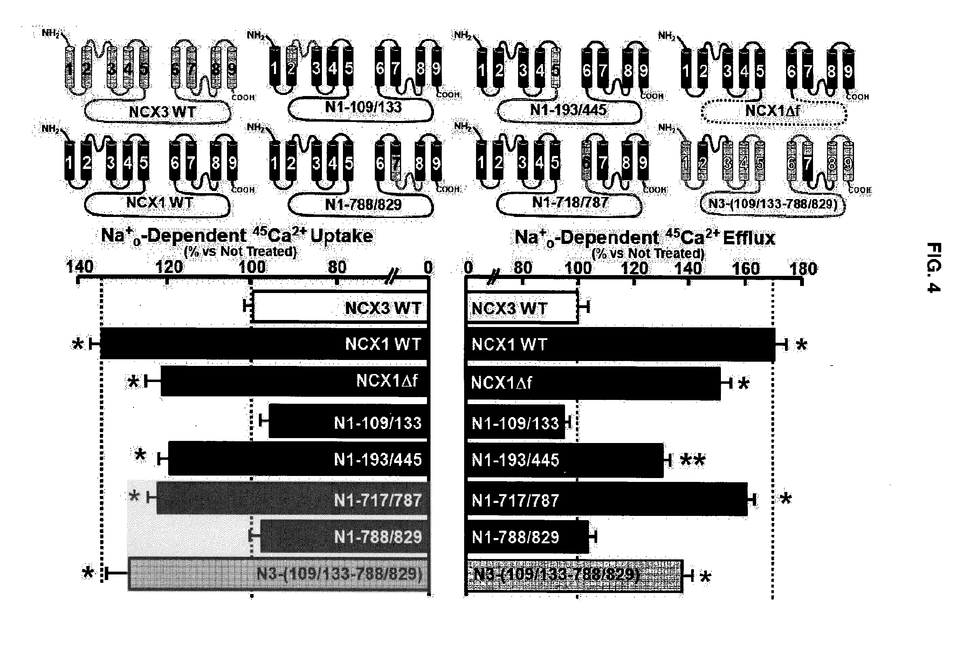

In order to establish the molecular determinants of the effect of neurounina-1 on NCX1, we firstly performed a Na⁺-dependent Ca²⁺ influx assay on BHK cells stably transfected with a mutated NCX1 exchanger (NCX1Δf), in which a large portion of the f loop, region 241-680, was removed by deletion mutagenesis. The activity of the mutant NCX1Δf exposed to 10 nM neurounina-1 was significantly increased compared to wild-type NCX3 exposed to the same treatment (Fig 4), suggesting that the f-loop is not a critical region for neurounina-1 interaction or activity.

To deeper investigate on the regions and amino acids present on NCX1 responsible for the interaction with neurounina-1 compound, we utilized chimeric and site-mutated proteins. In particular, we generate chimeric proteins with a large sequence of NCX1 and some regions of NCX3 protein. All chimeric proteins used for these experiments showed functional features very similar to the wild-type proteins from which they derivate. By using the chimeric technique it is possible to determine the importance of NCX1 regions by substituting with analogue segments of NCX3 protein. Results showed that the substitution of each NCX1 region corresponding to amino acids 109-133 (named α₁) and 788-829 (named α₂) removed the pharmacological effect of neurounina-1 (N1-109/133 and N1-788/829 chimeras) (Fig. 4). Moreover, the chimeric protein generated by NCX3 structure in which α₁ and α₂ regions were substituted with the corresponding NCX1 sequences showed a significantly increased activity by neurounina-1 compound (N3-(109/133-788/829) chimera) (Fig. 4). Therefore, these two regions result to be involved in the pharmacological modulation of NCX1 from neurounina-1 compound.

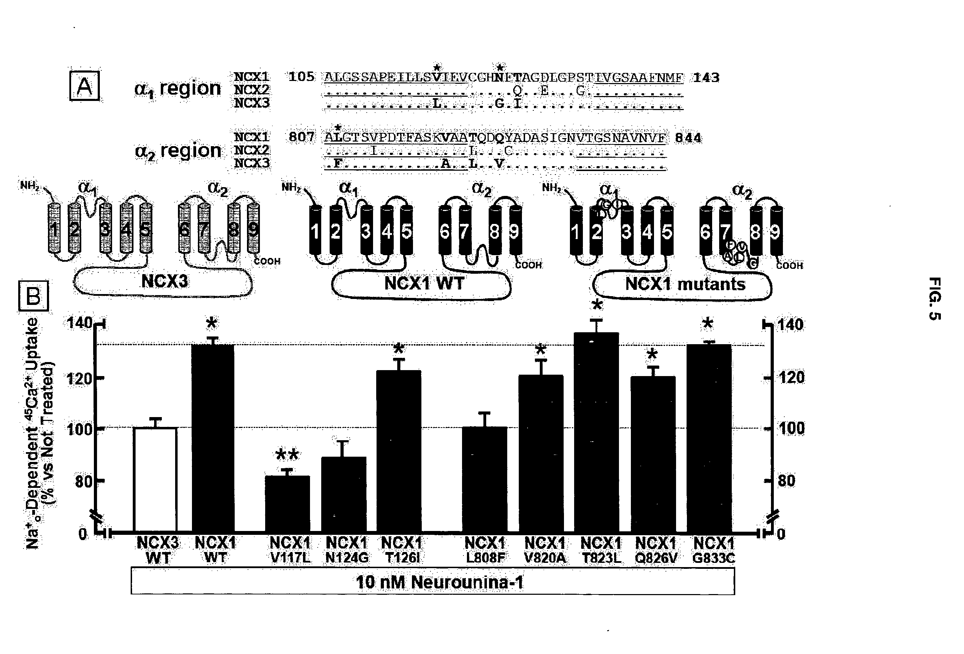

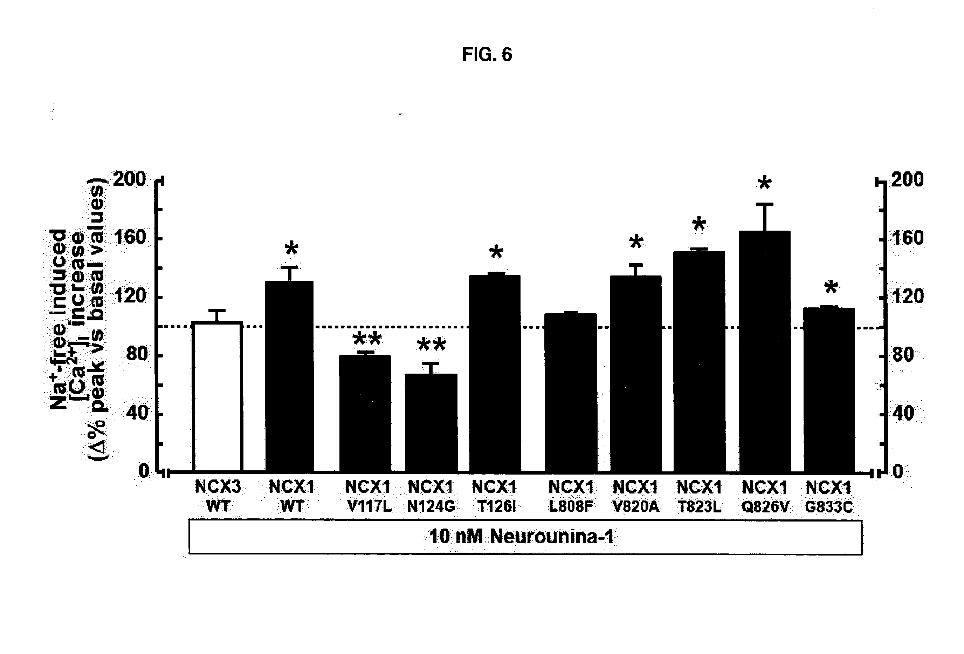

To identify the critical residues involved in drug sensitivity between NCX1 and NCX3 proteins, each residue in NCX1, in the regions α₁ and α₂, was substituted with the corresponding residue in NCX3. Results showed that the single mutation of valin 117 (V117L), asparagine 124 (N124G) and leucin 808 (L808F) generate a functional NCX1 exchanger that is insensible to neurounina-1 compound (Fig.5 and 6). Collected data suggest that these three amino acids are the molecular determinant requested to have the pharmacological effect of neurounina-1 on the exchanger.

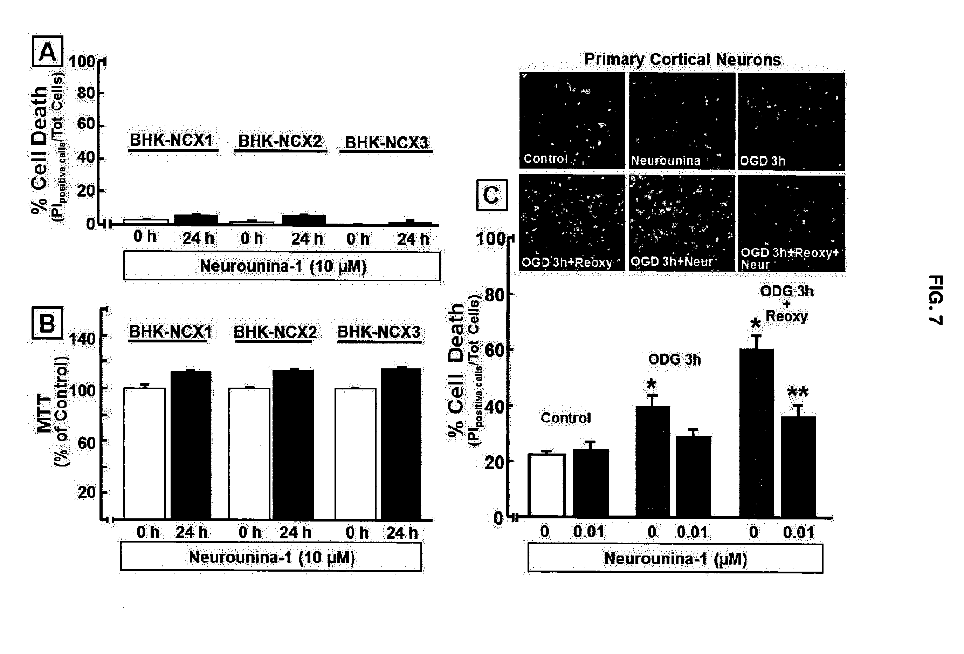

This data show that neurounina-1 increases NCX1 and NCX2 activity in both the forward and in reverse mode. The high stimulatory activity of neurounina-1 on NCX activity would be very useful in stroke treatment. Experiments, performed to evaluate the toxicity, showed that 10 µM of neurounina-1, a concentration almost 10.000 times greater than the EC₅₀, did not affect mitochondrial activity and cell viability (Fig. 7A).

Moreover, 10 nM of neurounina-1 reduced cell death of rat cortical neurons exposed to 3 hours of oxygen and glucose deprivation (OGD) followed by 21 hours of reoxygenation (Fig. 7B).

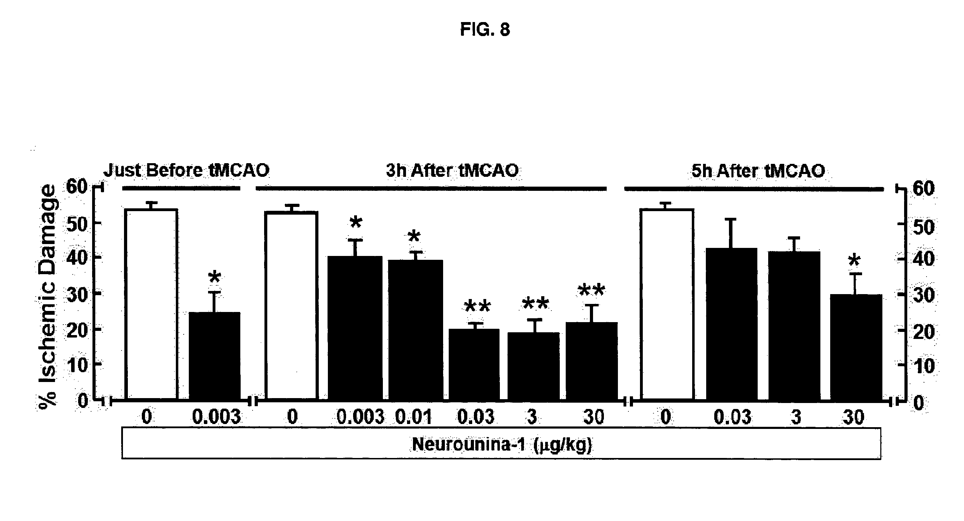

Consistently, in *in vivo* model of cerebral ischemia, the intraperitoneal administration of neurounina-1 decreased the ischemic volume after transient middle cerebral artery occlusion (tMCAO) insult. The effect of neurounina-1 showed not only dose-dependence, but also time-dependence from the time of pharmacological administration and of stroke induction. Neurounina-1 significantly reduced ischemic damage when administered just before tMCAO induction at doses of 3 ng/kg. This neuroprotective effect of neurounina-1 was still present if administrated intraperitoneally 3 hours after tMCAO at the dose-range between 3 ng/kg to 30 µg/kg. After five hours from tMCAO, neurounina-1 showed a neuroprotective effect at the minimal dose of 30 µg/kg (Fig. 8).

### EXPERIMENTAL SECTION

The synthetic procedure of the synthesis of 7-nitro-5-phenyl-1-(pyrrolidin-1-ylmethyl)-1*H-*benzo[e][1,4]diazepin-2(3*H*)-one and other benzodiazepine derivatives from formula (I) is illustrated as follow:

### 1.1 Synthesis of 7-nitro-5-phenyl-1-(pyrrolidin-1-ylmethyl)-1H-benzo[e][1,4]diazepin-2(3H)-one also defined as neurounina-1

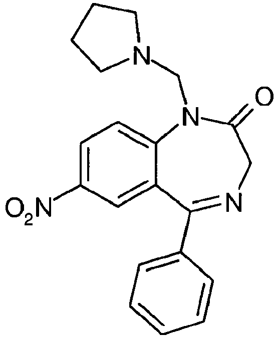

Formaldehyde (0.1 mL, 3.5 mmol) was added to pyrrolidine (0.3 mL, 3.5 mmol) at 0°C and the obtained solution was added to nitrazepam (0.1 g, 0.35 mmol), previously dissolved in glacial acetic acid (5 mL). The reaction mixture was placed in a closed reaction vessel, equipped with temperature control unit, and irradiated according to the following parameters: initial power, 500 W; initial time, 1 min (ramping); final power, 500 W; T 80 °C; reaction time, 15 min.

The reaction mixture was extracted with 2 N NaOH (3 x 40 mL) and isopropanol/chloroform (1/1, v/v, 40 mL). The organic phase was washed with brine, dried over anhydrous Na₂SO₄, filtered and concentrated *in vacuo.* The obtained residue was purified on a silica gel column eluted with dichloromethane/methanol (8/2, v/v) affording 61 mg of purified product (yield 48%) which was converted to the corresponding trifluoroacetate salt by dissolving in 0.1 % TFA in H₂O/acetonitrile (60/40, v/v); the obtained solution was frozen and lyophilized furnishing the desired salt. ESI-MS calcd for C₂₀H₂₀N₄O₃ 364.4 found [M+H]⁺ 365.1.

### 1.2 Synthesis of 7-amino-5-phenyl-1-(pyrrolidin-1-ylmethyl)-1H-benzo[e][1,4]diazepin-2(3H)-one

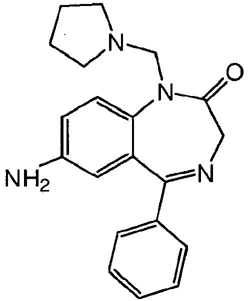

7-Nitro-5-phenyl-1-(pyrrolidin-1-ylmethyl)-1H-benzo[e][1,4]diazepin-2(3H)-one (0,1 g, 0,27 mmol), was dissolved in methanol (10 mL) and acetic acid (1 mL) and hydrogenated using 10% Pd/C (10 mg) as catalyst. The reaction mixture has been stirred at 3 atm until no more consumption of hydrogen was observed; then it was filtered and the solvent was removed *in vacuo*. The obtained residue was purified on a silica gel column eluted with dichloromethane/methanol (8/2, v/v) affording 78 mg of 7-amino-5-phenyl-1-(pyrrolidin-1-ylmethyl)-1H-benzo[e][1,4]diazepin-2(3H)-one (yield 87%) which was converted to the corresponding trifluoroacetate salt by dissolving in 0.1 % TFA in H₂O/acetonitrile (60/40, v/v); the obtained solution was frozen and lyophilized furnishing the desired salt. ESI-MS calcd for C₂₀H₂₂N₄O 334.4 found [M+H]⁺ 335.3.

### 1.3 Synthesis of 5-(2-chlorophenyl)-7-nitro-1-(pyrrolidin-1-ylmethyl)-1H-benzo[e][1,4]diazepin-2(3H)-one

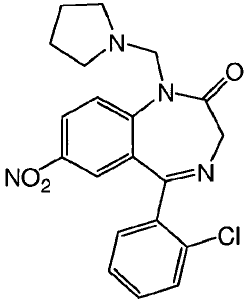

Starting from clonazepam and pyrrolidine, 5-(2-chlorophenyl)-7-nitro-1-(pyrrolidin-1-ylmethyl)-1H-benzo[e][1,4]diazepin-2(3H)-one has been obtained following the synthetic procedure reported for compound la: yield 67,8%. ESI-MS calcd for C₂₀H₁₉ClN₄O₃ 398,8 found [M + H]⁺400,5.

### 1.4 Synthesis of 7-chloro-5-phenyl-1-(pyrrolidin-1-ylmethyl)-1H-benzo[e][1,4]diazepin-2(3H)-one

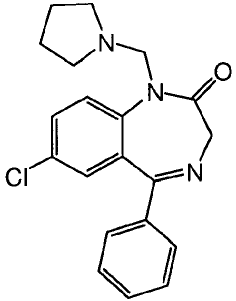

Starting from nordazepam and pyrrolidine, 7-chloro-5-phenyl-1-(pyrrolidin-1-ylmethyl)-1H-benzo[e][1,4]diazepin-2(3H)-one has been obtained following the synthetic procedure reported for compound la: yield 51,3%. ESI-MS calcd for C₂₀H₂₀ClN₃O 353,8 found [M + H]⁺ 354,6.

### 1.5 Synthesis of 7-chloro-5-(2-chlorophenyl)-3-hydroxy-1-(pyrrolidin-1-ylmethyl)-1H-benzo[e][1,4]diazepin-2(3H)-one

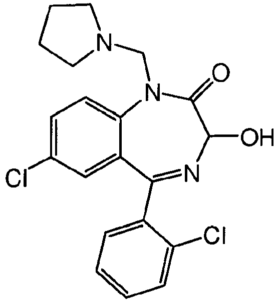

Starting from lorazepam and pyrrolidine, 7-chloro-5-(2-chlorophenyl)-3-hydroxy-1-(pyrrolidin-1-ylmethyl)-1H-benzo[e][1,4]diazepin-2(3H)-one has been obtained following the synthetic procedure reported for compound la: yield 61%. ESI-MS calcd for C₂₀H₁₉Cl₂N₃O₂ 404,3 found [M + H]⁺405,0.

### 1.6 Synthesis of 7-nitro-5-phenyl-1-(piperidin-1-ylmethyl)-1H-benzo[e][1,4]diazepin-2(3H)-one

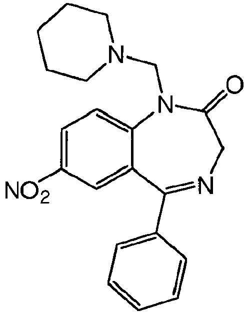

Starting from nitrazepam and piperidine, 7-nitro-5-phenyl-1-(piperidin-1-ylmethyl)-1H-benzo[e][1,4]diazepin-2(3H)-one has been obtained following the synthetic procedure reported for compound la: yield 56%. ESI-MS calcd for C₂₁H₂₂N₄O₃ 378,4 found [M+H]⁺379,3.

### 2. In vitro Studies

### Cellular cultures

The activity of NCX was established by means of three different subclones of baby hamsted kidney (BHK) cells that were stably transfected with one of each NCX isoforms, named BHK-NCX1, BHK-NCX2 and BHK-NCX3. These cell lines were grown on plastic dishes in a mix of DMEM and Ham's F12 media (1:1) (Gibco, Invitrogen, MI, Italy) supplemented with 5% fetal bovine serum, 100 U/ml penicillin, and 100 µg/ml streptomycin (Sigma, St. Louis, Missouri, USA). Cells were cultured in a humidified 5% CO₂ atmosphere and at the temperature of 37°C.

All experiments aimed to identify the binding site of neurounina-1 were performed on BHK cells stably transfected with a single NCX chimera or mutant.

The effect of neurounina-1 was evaluated on cell viability by using rat primary cortical neurons obtained as follow. Briefly, cortical neurons were prepared from brains of 1-2 day-old new-born rats, anaesthetizes with halothane in a glass chamber and decapitated. Dissection and dissociation were performed in a Ca²⁺-Mg²⁺-free PBS containing glucose. The brain was removed from the skull and the cerebral cortex was removed. Cerebral cortex was first digested enzymatically and after dispersed mechanically by trituration. Cellular suspension was plated on coverslips previously pre-coated with poly-D-lysine 1mg/ml and cultured in "Neurobasal" medium containing B27 (1:50) and Glutamax)1:100).

### Studies on the activity of NCX by means of the ⁴⁵Ca²⁺ radiotracer

The activity of the Na⁺/Ca²⁺ exchanger was measured by means of ⁴⁵Ca²⁺ radiotracer variations in the cytoplasm of cells exposed to a protocol stimulating the forward or the reverse mode of the antiporter. The BHK cells expressing each mutated or non-mutated NCX isoform were cultured in 12-well dishes and incubated in 145 mM NaCl for 10 minutes and then in a medium containing only 145 mM N-Methyl D-glucamine (NMDG) and 10 µM of ⁴⁵Ca²⁺. This rapid substitution of the medium from a high level of Na⁺ to another one without Na⁺ allow the ⁴⁵Ca²⁺ influx (reverse mode). Before the medium substitution cells was incubated with 1 mM ouabain, a Na⁺/K⁺ pump inhibitor, and 10 µM monensin, a ionophore for extracellular Na⁺. After 30 seconds incubation, cells were washed with an ice-cold solution containing 2 mM La³⁺ to stop ⁴⁵Ca²⁺ uptake. The medium was removed and cells were subsequently lysed with 0.1 N NaOH and aliquots were taken to determine radioactivity and protein content.

In order to measure NCX activity in the forward mode, cells were firstly loaded with ⁴⁵Ca²⁺ and subsequently exposed to a chemical protocol to stimulate the efflux of the radiotracer in the extracellular medium. In particular, cells were loaded with ⁴⁵Ca²⁺ by using a solution containing 1 µM ionomycin, 145 mM Na⁺ and ⁴⁵Ca²⁺. Next, cells were exposed to a Ca²⁺-free solution containing 2 mM EGTA. One µM thapsigargin, a Ca²⁺-ATPase inhibitor, was present in both solutions in order to prevent ⁴⁵Ca²⁺ influx in intracellular organelles. After 60 second, cells were lysed with 0.1 mM NaOH and aliquots were taken to determine cytoplasmatic radioactivity.

Cells were incubated for 30 minutes with different neurounina-1 concentrations.

### Electrophysiology

NCX currents (/_{NCX}) was recorded from BHK wild-type and BHK stably transfected cells with NCX1, NCX2, and NCX3 and all each mutated of single isoforrms by patch-clamp technique in whole-cell configuration. Records were performed at the temperature of 20-22 °C using a commercially available amplifier (Axopatch 200B, Molecular Devices, Sunnyvale, CA) using glass micropipettes with a resistance of 3.5 MΩ. Currents were filtered at 5 kHz and digitized by use of a Digidata 1322A interface (Molecular Devices, Sunnyvale, CA). Data were acquired and analyzed by use of pClamp software (version 9.0, Molecular Devices). In brief, /_{NCX} was recorded starting from a holding potential of -60 mV up to a short-step depolarization at +60 mV (60 ms) (He et al., 2003; Xiao et al., 2004). Then, a descending voltage ramp from +60 mV to -120 mV was applied. The current recorded in the descending portion of the ramp (from +60 to -120 mV) was used to plot the current-voltage (I-V) relation curve. The magnitudes of /_{NCX} were measured at the end of +60 mV (reverse mode) and at the end of -120 mV (forward mode), respectively. Because Ni²⁺ blocks I_{NCX}, NiCl₂ (5 mM) was routinely added to measure the NCX-independent currents. The Ni²⁺-insensitive components were subtracted from total currents to isolate I_{NCX}. External Ringer's solution contained 126 mM NaCl, 1.2 mM NaHPO₄, 2.4 mM KCI, 2.4 mM CaCl₂, 1.2 mM MgCl₂, 10 mM glucose, 18 mM NaHCO₃, 20 mM tetraethylammonium (TEA), 10 nM TTX, and 10 µM nimodipine, pH 7.4. The dialyzing pipette solution contained 100 mM potassium gluconate, 10 mM TEA, 20 mM NaCl, 1 mM Mg-ATP, 0.1 mM CaCl₂, 2 mM MgCl₂, 0.75 mM EGTA, 10 mM HEPES, adjusted to pH 7.2 with CsOH. TEA (20 mM) and Cs were included to block delayed outward rectifier K⁺ components, nimodipine (10 µM) and TTX (50 nM) were added to external solution to block L-type Ca channels and TTX-sensitive Na⁺ channels, respectively. /_{NCX} values were normalized for membrane capacitance as reported previously. Possible changes in cell size occurring upon specific treatments were calculated by monitoring the capacitance of each cell membrane, which is directly related to membrane surface area, and by expressing the current amplitude data as current densities (pA/pF). Capacitive currents were estimated by electrophysiological protocol (versione 9.0, Molecular Devices, Sunnyvale, CA, USA).

### Microfluorimetry

NCX activity has been measured considering the variation of intracellular concentrations of Ca²⁺ ions ([Ca²⁺]ᵢ) due to its activation by means of single-cell microfluorimetry. In particular Fura-2-acetoxymethyl ester (Fura-2AM) was used as [Ca²⁺]ᵢ fluorescent probe. This molecule is an ester moiety that, thanks to this modification, crosses cell membranes. Once inside the cell, the acetoxymethyl groups are removed by cellular esterases and remains localized within the cytoplasm, where it will measure the [Ca²⁺]ᵢ. Fura-2 alternatively illuminated by wavelengths at both 340 nm and 380 nm produces an emission at 510 nm. Considering its interaction with Ca²⁺ ions, Fura-2 can be excited at two different λ values. In particular, when Fura-2 interacts with Ca²⁺ ions can be excited only at 340 nm, whereas when ot is free the probe will be excited only at 380 nm. In both cases, this probe will produce an emission at 510 nm; the ratio between the two emission relative to 340 and 380 excitation allow to quantify [Ca²⁺]ᵢ in the time considering the calibration curve previously performed. Considering these parameters it will be possible to establish [Ca²⁺]ᵢ before and after the pharmacological treatment.

Briefly, BHK cells wild type and/or BHK trasnsfected with NCX mutants, grown on glass coverslips, were loaded with Fura-2 acetoxymethyl ester (Fura-2AM) (10 µM) for 30 minutes at 37°C in normal Krebs solution containing the following (in mM): 5.5 KCI, 160 NaCl, 1.2 MgCl₂, 1.5 CaCl₂, 10 glucose, and 10 Hepes-NaOH, pH 7.4. At the end of the Fura-2AM loading period, the coverslips were washed two times and than placed into a perfusion chamber (Medical System, Co. Greenvale, NY, USA) mounted onto the stage of an inverted Zeiss Axiovert 200 microscope (Carl Zeiss, Germany) equipped with a FLUAR 40x oil objective lens. The experiments were carried out with a digital imaging system composed of MicroMax 512BFT cooled CCD camera (Princeton Instruments, Trenton, NJ, USA), LAMBDA 10-2 filter wheeler (Sutter Instruments, Novato, CA, USA), and Meta-Morph/MetaFluor Imaging System software (Universal Imaging, West Chester, PA, USA). After loading, cells were alternatively illuminated at wavelengths of 340 nm and 380 nm by a Xenon lamp. The emitted light was passed through a 512 nm barrier filter. Fura-2 fluorescence intensity was measured every 3 seconds. Assuming that the K_{D} for Fura-2 was 224 nM, the equation of Grynkiewicz was used for calibration. NCX activity was evaluated as Ca2+ uptake through the reverse mode by switching the normal Krebs medium containing thapsigargi (1µM) to Na⁺-deficient NMDG+ medium (Na⁺-free) containing (in mM): 5.5 KCI, 147 N-Methyl glucamine, 1.2 MgCl₂, 1.5 CaCl₂, 10 glucose, and 10 Hepes-NaOH (pH 7.4). All the results are presented as percentage of cytosolic Ca²⁺ concentration increase above basal values and expressed as mean± S.E.M.

### Oxygen-glucose deprivation (OGD)

The oxygen and glucose deprivation (OGD) was used as a model to reproduce in vitro a condition similar to that observed in vivo when a cerebral vessel was occluded as it occurs in stroke. Briefly, cells were incubated in a medium oxygen and glucose-free and containing: NaCl 116 mM, KCI 5.4 mM, MgSO₄ 0.8 mM, NaHCO₃ 26.2 mM, NaH₂PO₄ 1 mM, CaCl₂ 1.8 mM, glycin 0.01 mM e phenol 0.001 mM (w/v) (Molinaro et al., 2008). This medium was previously saturated with 1 atm of a gas containing 95% N₂ and 5% CO₂ for 20 minutes. In order to avoid the reoxygenation of the medium by the atmospheric oxygen, cells were placed in a chamber for anaerobiosis in which air was saturated with a gas containing 95% N₂ and 5% CO₂ for 10 minutes and maintained at 37°C for 3 hours (Scorziello et al., 2001). In this experimental conditions a significantly reduction of pO2 in the medium occurred. At the end of OGD, pO₂ levels in the medium were reduced by 30%. At the end of OGD the cells were exposed to 24 hr reoxygenation obtained by changing the medium with one containing glucose and O₂ and maintaining the cells in normoxic condition in presence of 5% CO₂ e 95% air. This procedure allow to reproduce in vitro a condition similar to that observed in vivo after the restoration of cerebral blood flow. The assessment of neuronal damage was performed either after 3 hrs of OGD or after 24 hrs reoxygenation.

### Evaluation of cell viability

Mitochondrial dysfunction was evaluated by measuring mitochondrial dehydrogenase activity in BHK cells exposed to 10 mM of neurounina-1 for 24 hours. 3[4,5-dimethylthiazol-2-y1]-2,5-diphenyltetrazolium bromide (MTT) was used as substrate for these enzymes. Mitochondrial dehydrogenases reduce MTT to purple formazan that is water-insoluble and can be measured by a spectrophotometer at a wavelength of 540 nm. At the end of the incubation, the cell medium was removed and the cells were incubated with 2 ml of a PBS solution containing MTT at the concentration of 0,5 mg/ml for one hour at 37°C in an atmosphere of 5% of CO₂. At the end of the incubation, the water-insoluble formazan is dissolved in 1 ml of DMSO, and measured by a spectrophotometer at a wavelength of 540 nm.

Fluorescein diacetate (FDA) is an apolar ester that could cross plasmamembrane. When FDA is hydrolysed into the cell by endocellular esterase it shows a fluorescent green colour. The polar compound propidium-iodide (PI) can not cross an intact plasmamembrane, so this is a good marker of necrotic cells. Once PI enters into the cell, this compound interact with nuclear DNA and give a fluorescent red light. Consequently, in the same cellular population, it is possible to discriminate live cells from dead cells, because the last group are PI-positive. In PI/PDA experiments, BHK cells were firstly treated with 10 µM neurounina-1 for 24 hours and then incubated for 3 minutes, at 22°C, in a PBS solution containing fluorescein diacetate (FDA; Sigma) and propidium iodide (PI, Calbiochem, San Diego, CA, U.S.A.), at the concentrations of di 36 µM e 7 µM, respectively. At the end of the incubation, cells were washed with PBS and observed at the microscope.

### 3. In vivo Studies

### Cerebral Ischemia

For experimental stroke male wild-type C57BL of 2 months have been used. All mice were anesthetized with a gas containing 70 % N₂O, 28% O₂ e 2% sevoflurane).

Usually, after anesthesia an incision on the ventral neck is made and, under an operating stereo-microscope and with the help of microsurgery twizzers, the muscle is diverted until the common carotid artery (CCA). Then, through smoothly diversion, the CCA is isolated from the vague nerve and the jugulars vein. Analogously, the external carotid artery (ECA) and the internal carotid artery (ICA) are isolated as well.After the isolation of these vessels and the positioning of vascular microclips, a nylon filament (5-0, Ethilon, 11 mm length) is inserted into the internal carotid artery (ICA), at the level of bifurcation with the common carotid artery. The filament is pushed in cranial direction until is reached the bifurcation of the middle cerebral artery (MCA), whose flux is interrupted. After the insertion of the filament the wound is closed with a temporary suture and the mouse is let to awake. After 60 minutes the animal is re-anesthesized and the ischemia is interrupted through the filament rimotion from the vessel. The reduction in cerebral blood flow has been evaluated by means of a cerebral laser-Doppler system (Periflux, Sweden with software Perimed 1.30) that uses a probe fixed on the skull. The body temperature has been maintained constant by means of a homoeothermic blanket connected to a rectal probe. Few minutes after the end of the surgical procedure the mouse awakes and, on the nasis of the pain degree noticed, is decided for the administration of analgesic drugs. Neurounina-1 administration has been done intraperitoneally at different dosages, immediately before, 3 hours or 5 hours the ischemia onset. 24 hours after the surgical procedure, the animal is killed through cervical dislocation and the brain is removed from the skull and analyzed for the determination of the ischemic damage extension. In particular, by means of a vibratome, serial coronal brain sections of 500 mm are obtained. These brain sections are treated with 2,3,5-triphenyl tetrazolium chloride (TTC), an organic compound that has the peculiarity of being oxidized and precipitated as formazan salt only from vital tissue. By contrast, the ischemic tissue, being not able to generate this reaction, remains white. After that, with the help of a image analyzer connect to a PC, the area of the infarcis determined by delimiting the surface of the brain slice that does not take up the red color after TTC treatment. The software used (Image Pro-plus 4.1) allows, then, to obtain the brain infarct volume from the value of the infarct areas obtained in close brain slices.Data obtained in this manner are then elaborated to obtain a percentage evaluation of the ischemic damage induced compared to the brain hemisphere ipsilateral to the ischemic lesion.

## Claims

1. Compounds of formula (I): 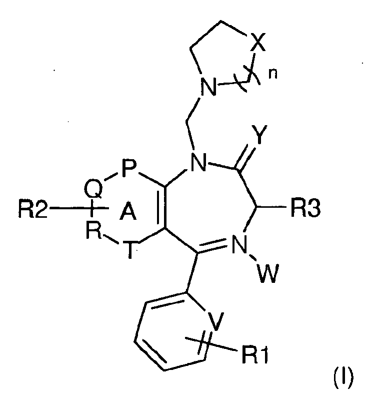 wherein
ring A is a 5- or 6-membered aromatic or heteroaromatic ring, and therefore a group chosen from P, Q, R or T may be absent; P, Q, R and T independently of one another are chosen from CH, N, O and S;
R1 and R2 independently of one another are chosen from hydrogen, halogen, Ak, O-Ak, Ph, O-Ph, Ph-Ak, heteroaryl, CF₃, OCF₃, NO₂, NH₂, NH-Ak, NAk₂, COOAk, S-Ak, S02-Ak, CN;
R3 is chosen from hydrogen, halogen, Ak, OAk, O-CO-Ak, OH;
Y is chosen from O and S;
W is O, if present;
V is chosen from CH or C if substituted by R1, and N;
X is chosen from CH2, O, NH, NAk and n = 1,2,3;
wherein Ak means alkyl;
in the compounds of formula (I), optical isomers, enantiomers or diastereoisomers and mixtures thereof, and pharmaceutically acceptable organic and inorganic salts are intended to be included.

2. Compounds of formula (I) according to claim 1, wherein
X is CH2 and n is 1 or 2;
ring A, R1, R2, R3, Y, V and W are as defined in claim 1;
or
ring A is benzene;
R2 is CI, NO₂ or NH₂;
X, R1, R3, Y, V and W are as defined in claim 1;
or
V is CH or C if substituted by R1;
R1 is H or Cl;
X, A, R2, R3, Y, and W are as defined in claim 1;
or
R3 is H or OH;
X, A, R1, R2, Y, V and W are as defined in claim 1;
or
Y is O;
X, A, R1, R2, R3, V and W are as defined in claim 1;
or
W is absent;
X, A, R1, R2, R3, Y and V are as defined in claim 1.

3. Compounds of formula (I) according to claim 2, wherein
X is CH2 and n is 1 or 2;
ring A is benzene;
R1 is H or Cl;
R2 is Cl, NO₂ or NH₂;
R3 is H or OH;
Y is O;
V is CH or C, if substituted by R1;
W is absent.

4. Compounds according to claim 3 of formula (Ia) or (Ib):

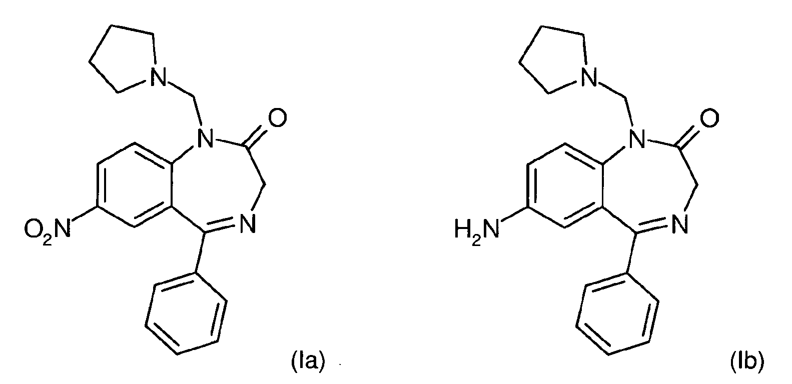

5. Compounds of formula (I) according to any one of the claims 1-4 for medical use.

6. Compounds of formula (I) according to any one of the claims 1-4 for use as activator of NCX1 and NCX2.

7. Compounds of formula (I) according to any one of the claims 1-4 for use in the treatment of cerebral ischemia or any other disease **characterized by** the loss of sodium and calcium ions homeostasis control, such as amyotrophic lateral sclerosis, Alzheimer's disease, Parkinson's disease, renal and cardiac ischemia, and other neurodegenerative diseases.

8. Compounds of formula (I) according to any one of the claims 1-4 for use in the treatment of anxiety, sedation, insomnia and striated muscle spasms.

9. Pharmaceutical compositions comprising at least one compound of formula (I) according to any one of the claims 1-4, and at least another pharmaceutically acceptable ingredient.

10. Process for synthesizing a compound of formula (I) according to any one of the claims 1-4, said process comprising a reaction of a compound of formula (II) 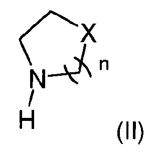 wherein X is chosen from CH₂, O, NH, NAk and n = 1,2,3;
with formaldehyde;
the mixture so obtained is then reacted with a compound of formula (III): 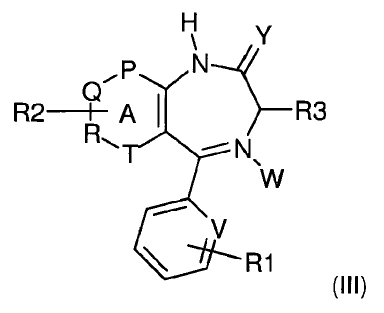 wherein
ring A is a 5- or 6-membered aromatic or heteroaromatic ring, and therefore a group chosen from P, Q, R or T may be present; P, Q, R and T independently of one another are chosen from CH, N, O and S;
R1 and R2 independently of one another are chosen from hydrogen, halogen, Ak, O-Ak, Ph, O-pH, Ph-Ak, heteroaryl, CF₃, -OCF₃, NO₂, NH₂, NH-Ak, NAk₂, COOAk, S-Ak, S02-Ak, CN;
R3 is chosen from hydrogen, halogen, Ak, OAk, O-CO-Ak, OH;
Y is chosen from O and S;
W is O, if present;
V is chosen from CH or C if substituted by R1, and N;
wherein Ak means alkyl.

## Patentansprüche

1. Verbindungen gemäß der Formel (I): 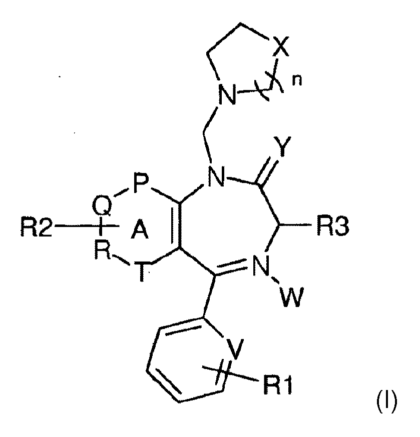 worin
der Ring A ein 5- oder 6-gliedriger aromatischer oder heteroaromatischer Ring ist und daher eine Gruppe ausgewählt aus P, Q, R oder T abwesend sein kann, wobei P, Q, R und T unabhängig voneinander aus CH, N, O und S ausgewählt sind,
R1 und R2 unabhängig voneinander aus Wasserstoff, Halogen, Ak, O-Ak, Ph, O-Ph, Ph-Ak, Heteroaryl, CF₃, OCF₃, NO₂, NH₂, NH-Ak, NAk₂, COOAK, S-Ak, S02-Ak, CN ausgewählt sind,
R3 aus Wasserstoff, Halogen, Ak, OAk, O-CO-Ak, OH ausgewählt ist,
Y aus O und S ausgewählt ist,
W, falls anwesend, O ist,
V aus CH oder C, wenn mit R1 substituiert, und N ausgewählt ist,
X aus CH2, O, NH, NAk ausgewählt ist und n = 1, 2 oder 3 ist,
wobei Ak Alkyl bedeutet,
wobei es beabsichtigt ist, dass in den Verbindungen gemäß der Formel (I) optische Isomere, Enantiomere oder Diastereoisomere und Mischungen hiervon sowie pharmazeutisch akzeptable organische und anorganische Salze eingeschlossen sind.

2. Verbindungen gemäß der Formel (I) nach Anspruch 1, wobei
X CH2 ist und n 1 oder 2 ist,
der Ring A, R1, R2, R3, Y, V und W wie in dem Anspruch 1 definiert sind,
oder
der Ring A Benzol ist,
R2 Cl, NO₂ oder NH₂ ist,
X, R1, R3, Y, V und W wie in dem Anspruch 1 definiert sind,
oder
V CH oder C, wenn mit R1 substituiert, ist,
R1 H oder Cl ist,
X, A, R2, R3, Y und W wie in dem Anspruch 1 definiert sind,
oder
R3 H oder OH ist,
X, A, R1, R2, Y, V und W wie in dem Anspruch 1 definiert sind,
oder
Y O ist,
X, A, R1, R2, R3, V und W wie in dem Anspruch 1 definiert sind,
oder
W abwesend ist,
X, A, R1, R2, R3, Y und V wie in dem Anspruch 1 definiert sind.

3. Verbindungen gemäß der Formel (I) nach Anspruch 2, wobei
X CH2 ist und n 1 oder 2 ist,
der Ring A Benzol ist,
R1 H oder Cl ist,
R2 Cl, NO₂ oder NH₂ ist,
R3 H oder OH ist,
Y O ist,
V CH oder C, wenn mit R1 substituiert, ist,
W abwesend ist.

4. Verbindungen nach Anspruch 3 gemäß der Formel (la) oder (Ib):

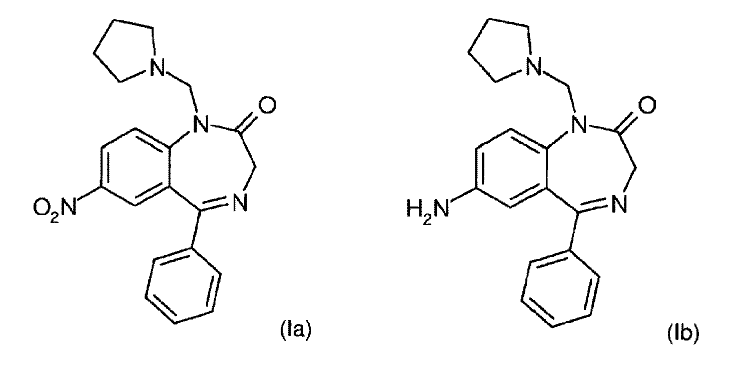

5. Verbindungen gemäß der Formel (I) nach einem der Ansprüche 1 bis 4 zur medizinischen Verwendung.

6. Verbindungen gemäß der Formel (I) nach einem der Ansprüche 1 bis 4 zur Verwendung als Aktivator von NCX1 und NCX2.

7. Verbindungen gemäß der Formel (I) nach einem der Ansprüche 1 bis 4 zur Verwendung bei der Behandlung von zerebraler Ischämie oder einer anderen Erkrankung **gekennzeichnet durch** den Verlust von Natrium- und Calciumion-Homöostasesteuerung, wie beispielsweise amyotropher Lateralsklerose, Alzheimer-Erkrankung, Parkinson-Erkrankung, renaler und kardialer Ischämie und anderer neurodegenerativer Erkrankungen.

8. Verbindungen gemäß der Formel (I) nach einem der Ansprüche 1 bis 4 zur Verwendung bei der Behandlung von Anststörung, Sedation, Schlafstörung und Spasmen der gestreiften Muskulatur.

9. Pharmazeutische Zusammensetzungen enthaltend wenigstens eine Verbindung gemäß der Formel (I) nach einem der Ansprüche 1 bis 4 und wenigstens einen anderen pharmazeutisch wirksamen Bestandteil.

10. Verfahren zum Synthesizieren einer Verbindung gemäß der Formel (I) nach einem der Ansprüche 1 bis 4, wobei das Verfahren die Reaktion einer Verbindung gemäß der Formel (II) 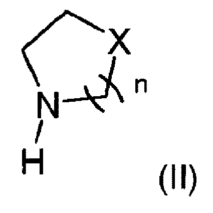 worin X aus CH₂, O, NH, NAk ausgewählt ist und n = 1, 2 oder 3 ist,
mit Formaldehyd, umfasst,
wobei die so erhaltene Mischung dann mit einer Verbindung gemäß der Formel (III) reagiert wird: 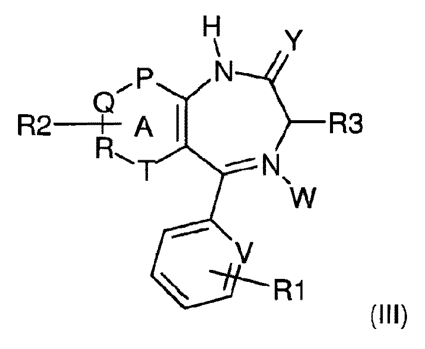 worin
der Ring A ein 5- oder 6-gliedriger aromatischer oder heteroaromatischer Ring ist und daher eine Gruppe ausgewählt aus P, Q, R oder T anwesend sein kann, wobei P, Q, R und T unabhängig voneinander aus CH, N, O und S ausgewählt sind,
R1 und R2 unabhängig voneinander aus Wasserstoff, Halogen, Ak, O-Ak, Ph, O-pH, Ph-Ak, Heteroaryl, CF₃, -OCF₃, NO₂, NH₂, NH-Ak, NAk₂, COOAk, S-Ak, S02-Ak, CN ausgewählt sind,
R3 aus Wasserstoff, Halogen, Ak, OAk, O-CO-Ak, OH ausgewählt ist,
Y aus O und S ausgewählt ist,
W, falls anwesend, O ist,
V aus CH oder C, wenn mit R1 substituiert, und N ausgewählt ist,
wobei Ak Alkyl bedeutet.

## Revendications

1. Composés de formule (I) : 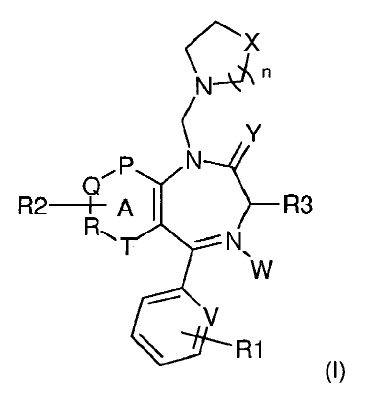 dans laquelle
le cycle A est un cycle aromatique ou hétéroaromatique de 5 ou 6 chaînons, et par conséquent un groupe choisi parmi P, Q, R ou T peut être absent ; P, Q, R et T indépendamment les uns des autres sont choisis parmi CH, N, O et S ;
R1 et R2 indépendamment l'un de l'autre sont choisis parmi un atome d'hydrogène, d'halogène, les groupes Ak, O-Ak, Ph, O-Ph, Ph-Ak, hétéroaryle, CF₃, OCF₃, NO₂, NH₂, NH-Ak, NAk₂, COOAk, S-Ak, SO2-Ak, CN ;
R3 est choisi parmi un atome d'hydrogène, d'halogène, les groupes Ak, OAk, O-CO-Ak, OH ;
Y est choisi parmi 0 et S ;
W représente O, si présent ;
V est choisi parmi CH ou C si substitué par R1, et N ;
X est choisi parmi CH2, O, NH, NAk et n = 1, 2, 3 ;
où Ak signifie alkyle ;
dans les composés de formule (I), leurs isomères optiques, énantiomères ou diastéréomères et mélanges, et des sels organiques et inorganiques pharmaceutiquement acceptables sont prévus être compris.

2. Composés de formule (I) selon la revendication 1, dans lesquels
X représente CH2 et n vaut 1 ou 2 ;
le cycle A, R1, R2, R3, Y, V et W sont tels que définis dans la revendication 1 ;
ou
le cycle A représente le benzène ;
R2 représente Cl, NO₂ ou NH₂ ;
X, R1, R3, Y, V et W sont tels que définis dans la revendication 1 ;
ou
V représente CH ou C si substitué par R1 ;
R1 représente H ou Cl ;
X, A, R2, R3, Y, et W sont tels que définis dans la revendication 1 ;
ou
R3 représente H ou OH ;
X, A, R1, R2, Y, V et W sont tels que définis dans la revendication 1 ;
ou
Y représente O ;
X, A, R1, R2, R3, V et W sont tels que définis dans la revendication 1 ;
ou
W est absent ;
X, A, R1, R2, R3, Y et V sont tels que définis dans la revendication 1.

3. Composés de formule (I) selon la revendication 2, dans lesquels
X représente CH2 et n vaut 1 ou 2 ;
le cycle A représente le benzène ;
R1 représente H ou Cl ;
R2 représente Cl, NO₂ ou NH₂ ;
R3 représente H ou OH ;
Y représente O ;
V représente CH ou C, si substitué par R1 ;
W est absent.

4. Composés selon la revendication 3 de formule (Ia) ou (Ib) :

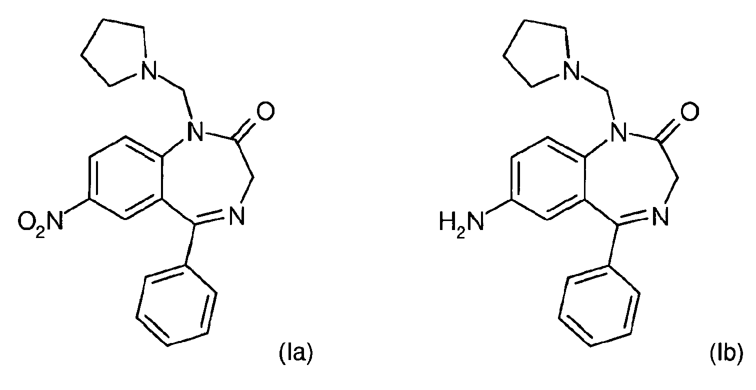

5. Composés de formule (I) selon l'une quelconque des revendications 1 à 4, pour une utilisation médicale.

6. Composés de formule (I) selon l'une quelconque des revendications 1 à 4, pour une utilisation en tant qu'activateur de NCX1 et NCX2.

7. Composés de formule (I) selon l'une quelconque des revendications 1 à 4, pour une utilisation dans le traitement de l'ischémie cérébrale ou de toute autre maladie **caractérisée par** la perte du contrôle de l'homéostasie des ions sodium et calcium, comme la sclérose latérale amyotrophique, la maladie d'Alzheimer, la maladie de Parkinson, l'ischémie rénale et cardiaque, et d'autres maladies neurodégénératives.

8. Composés de formule (I) selon l'une quelconque des revendications 1 à 4, pour une utilisation dans le traitement de l'anxiété, de la sédation, de l'insomnie et des spasmes des muscles striés.

9. Compositions pharmaceutiques comprenant au moins un composé de formule (I) selon l'une quelconque des revendications 1 à 4, et au moins un autre composant pharmaceutiquement acceptable.

10. Procédé de synthèse d'un composé de formule (I) selon l'une quelconque des revendications 1 à 4, ledit procédé comprenant une réaction d'un composé de formule (II) 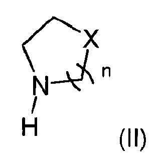 dans laquelle X est choisi parmi CH₂, 0, NH, NAk et n = 1, 2, 3 ;
avec du formaldéhyde ;
le mélange ainsi obtenu est ensuite mis à réagir avec un composé de formule (III) : 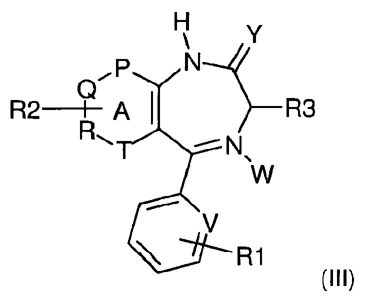 dans laquelle
le cycle A est un cycle aromatique ou hétéroaromatique de 5 ou 6 chaînons, et par conséquent un groupe choisi parmi P, Q, R ou T peut être présent ; P, Q, R et T indépendamment les uns des autres sont choisis parmi CH, N, O et S ;
R1 et R2 indépendamment l'un de l'autre sont choisis parmi un atome d'hydrogène, d'halogène, les groupes Ak, O-Ak, Ph, O-Ph, Ph-Ak, hétéroaryle, CF₃, -OCF₃, NO₂, NH₂, NH-Ak, NAk₂, COOAk, S-Ak, SO2-Ak, CN ;
R3 est choisi parmi un atome d'hydrogène, d'halogène, les groupes Ak, OAk, O-CO-Ak, OH ;
Y est choisi parmi 0 et S ;
W représente 0, si présent ;
V est choisi parmi CH ou C si substitué par R1, et N ;
où Ak signifie alkyle.
